# EUROPEAN PATENT SPECIFICATION

(11) **EP 0 930 316 B1**
(45) Date of publication and mention of the grant of the patent: **06.05.2004**
(21) Application number: 98310267.4
(22) Date of filing: 15.12.1998
(51) Int. Cl.: C07K 16/02, C07K 16/04, C07K 16/10, C07K 16/12, A61K 39/42, A61K 39/44

(54) **Oral product for the prevention and treatment of infectious gastroenteritides in calves**
Oralprodukt zur Prävention und Behandlung von ansteckender Gastroenteritis in Kälbern
Produit oral pour la prévention et le traitement des gastroentérites infectieuses des veaux

(30) Priority: 19.01.1998 CZ 15898
(43) Date of publication of application: 21.07.1999
(73) Proprietor: Medipharm CZ, s.r.o., 693 01 Hustopece u Brna (CZ)
(72) Inventor: Mican, Petr, 693 01 Hustopece u Brna (CZ); Stepanek, Jan, 592 56 Zvole nad Perstynem (CZ)
(74) Representative: McCallum, Graeme David

(56) References cited:
- WO-A-94/21284
- WO-A-98/54226
- ERHARD M H ET AL: "[New possibilities in oral immunoprophylaxis of newborn diarrhea in calves--a field study using specific egg antibodies ]. Neue Moglichkeiten in der oralen Immunprophylaxe der Neugeborenendiarrhoe des Kalbes--ein Feldversuch mit spezifischen Eiantikorpern." BERLINER UND MUNCHENER TIERARZTLICHE WOCHENSCHRIFT, (1993 NOV) 106 (11) 383-7. JOURNAL CODE: 9Q8. ISSN: 0005-9366., XP002102662 GERMANY: Germany, Federal Republic of
- DATABASE BIOSIS BIOSCIENCES INFORMATION SERVICE, PHILADELPHIA, PA, US KRDZALIC P ET AL: "NEWLY DEVELOPED IMMUNOPROPHYLAXIS OF BEEF GASTROENTERITIC DISEASES USING POLYVALENT VIRAL-BACTERIAL VACCINE LACTOVAC." XP002102665 & VET GLAS, (1990) 44 (6), 449-454. CODEN: VEGLAI. ISSN: 0350-2457.,
- ERHARD M H ET AL.: "The lipopeptide, Pam(3)Cys-Ser-(Lys) (4): An alternative adjuvant to Freund's adjuvant for the immunisation of chicken to produce egg yolk antibodies" ATLA- ALTERNATIVES TO LABORATORY ANIMALS, vol. 25, no. 2, March 1997, pages 173-181, XP002102663 ENGLAND
- REILLY R M ET AL.: "Oral delivery of antibodies - future pharmacokinetic trends" CLINICAL PHARMACOKINETICS, vol. 32, no. 4, April 1997, pages 313-323, XP002102664
- J. Vet. Med., B33, 1986, pg.: 241-252
- Zentralblatt für Hygiaene und Umweltmedizin (Int. J. Hygiene and Environm. Medicine), 1991, vol. 191, pg.: 277-301
- DATABASE WPI Week 199014, Derwent Publications Ltd., London, GB; Class B04, AN 1990-103166 & JP 2 053 737 A (YAMAMOTO T.) 22 February 1990

## Description

The invention pertains to an oral product for the prevention and treatment of infectious gastroenteritis of calves.

infections of the gastrointestinal tract rank among the most serious infectious diseases of young calves. Outbreaks are massive and particularly affect calves in the early postnatal period, i.e. at the age of ten to fourteen days.
The range of causative agents is rather wide, but a major role is played by bovine rotavirus, bovine coronavirus and enterotoxigenic strains of *Escherichia coli* (referred to as ETEC henceforth). All these agents induce local infections limited to the mucosa of the small intestine. The infection route is oral and the source of the agents is the environment that is usually heavily contaminated.

Rotaviruses and coronaviruses penetrate into superficial cells of the intestinal mucosa and propagate there, vigorously inducing degeneration and desquamation of enterocytes with the subsequent denudation and atrophy of the stroma of intestinal villi. ETEC adhere firmly to the surface of the intestinal mucosa and produce enterotoxin blocking entirely the function of the intestinal epithelial cells. The infection results in a loss of absorptive and metabolic potential of the intestinal mucosa, dysbacteriosis and hyperbiosis of the intestinal contents, transfer of tissue fluids into the intestinal lumen and general impairment of homeostasis. Clinical signs of the infection include moderate to severe diarrhoea, lethargy, dysorexia or anorexia, fever, dehydration and weight loss. Considerable economic losses result from growth retardation, high treatment costs and, last but not least, high mortality.

The approach to the protection against bacterial and viral infections of the gastrointestinal tract is determined by their local character. The most effective protection can be provided by specific antibodies to bovine rotavirus, bovine coronavirus and ETEC if present in time and in a sufficient concentration in the intestinal contents and on the surface of the small intestinal mucosa. The specific antibodies neutralize the potential of the agents to induce infection and prevent their adhesion onto the surface of the mucosa and penetration of rotaviruses and coronaviruses into epithelial cells of the intestinal mucosa. Some strains of lactacidogenic bacteria (*Enterococcus* spp, *Lactobacillus* spp.) are known to prevent the adhesion of pathogenic bacteria to the surface of intestinal cells and to inhibit, on a competitive basis, their propagation in the intestinal contents. Calves reared under natural conditions receive the specific antibodies from colostrum that is also the source of bacteria providing a favourable microbial environment in the intestine. One of the incidental effects of implementation of advanced technologies in cattle husbandry are variations in the composition and concentration of colostral antibodies resulting in an impairment of natural protection of calves against gastrointestinal infections.

A wide range of preventive and therapeutic methods have been implemented to enhance the resistance of calves to diarrhoeic diseases and to reduce economic losses resulting therefrom.

Thus the formation of antibodies shed in colostrum is stimulated by immunization of dams with antigens of the three major causative agents. However, antibodies present in blood at the time of immunization can partly or completely neutralize the administered antigens and thus to reduce or even eliminate the expected immunization effect. Moreover, relevant concentrations of specific antibodies persist in colostrum only during the first 24 to 48 hours after parturition and, consequently, the local passive immunity of the gastrointestinal tract is limited to the first three days of life.

Parenteral administration of specific antibodies to calves is almost ineffective, because the infection is limited to the small intestinal lumen, i.e. a place that is not accessible to parenterally administered antibodies.

Attempts were also made to protect calves by repeated oral administration of specific immunoglobulins prepared from colostrum of immunized cows. However, the respective preparative methods are rather sophisticated and expensive and the efficacy of the specific immunoglobulins is insufficient in calves suffering from gastrointestinal infections manifested by diarrhoea of various severity and other symptoms. Therefore, this method has not found a wider application.

Also unsuccessful were the attempts to treat calves with antibodies prepared from blood serum of immunized animals. Orally administered serum immunoglobulins are rapidly degraded by gastric and intestinal enzymes.

Antibiotic therapy of infectious gastroenteritis in calves is rather difficult. Antibiotics are ineffective against rotavirus and coronavirus infections and can only inhibit the propagation of ETEC in the intestine provided that the calves have been infected by a sensitive strain. Moreover, the administration of antibiotics results in unwanted changes in the composition of the normal intestinal microbial flora, is often followed by a rapid development of resistance of the causative agent, and can suppress the activity of the immune system of the patients.

Favourable results have been reported in calves treated for infectious gastroenteritis with rehydration solutions. Although such solutions have no direct effect on the infection, they alleviate incidental signs resulting from dehydration and loss of electrolytes and reduce the mortality rate.

Probiotic products suppress the adherence and propagation of enteropathogenic and enterotoxigenic bacteria in the intestine, support the restoration of normal intestinal biocenosis and thus enhance the natural resistance of the patient to enteric infections. However, probiotics cannot influence intestinal infections induced by rotaviruses and coronaviruses.

Other prior art includes Vet. Glas., vol. 44(6), 1990, p. 449-454 (Krdzalic, P. *et al*.)which discloses the immunoprophylaxis of beef gastroenteritic diseases using the polyvalent viral-bacterial baccine "Lactovac". Erhard MH *et al*. (Berl Munch Tierarztl Wochenschr. 1993 Nov;106(11):383-7) discloses oral immunoprophylaxis of neonatal calves using egg powder containing antibodies specific for ETEC and rotavirus. WO 94/21284 discloses the treatment of gastrointestinal disease in animals by the use of antibodies which have been pre-treated with a proteolytic enzyme or which are administered together with a proteolytic enzyme. JP 02-053737 teaches a process for the preparation of specific antibodies from eggs by vaccination of egg-laying hens with viral and bacterial antigens.

The drawbacks of the conventional therapeutic methods can be eliminated to a considerable extent by treatment with the oral product of the present invention.

According to the present invention there is provided an oral product for the prevention and therapy of infectious gastroenteritis in calves characterised in that it comprises antibodies to bovine rotavirus, bovine coronavirus and enterotoxigenic strains of Escherichia coli contained in colostrum of immunised cows and/or egg yolks of immunised hens and additionally comprising stabilised live culture of lactacidogenic bacteria, said oral product having a minimum titre of antibodies to rotavirus of 64/100 TCID₅₀ and a minimum agglutination titre of antibodies to enterotoxigenic strains of Escherichia coli of 80 per 10⁸ inactivated bacterial cells per 1 g, the concentration of the stabilised live cultures of lactacidogenic bacteria being within the range of 5 x 10⁵ to 50 x 10⁹ viable bacterial cells per 1 g.

Thus the specific passive immunotherapy is combined with the non-specific prophylactic effect of the probiotics. The active components of the product include (a) specific antibodies to bovine rotavirus and coronavirus and ETEC that can neutralize these agents in the intestinal tract of calves by preventing their adhesion and blocking their penetration into intestinal cells and thus interrupting or alleviating the course of the infection, and (b) stabilized live cultures of lactacidogenic bacteria, for example *Enterococcus* spp and/or *Lactobacillus* spp. which inhibit, on a competitive basis, the adherence to and propagation of enteropathogenic and enterotoxigenic bacteria in the intestinal tract, thus reducing the risk of bacterial sepsis, supporting the maintenance or restoration of microbial eubiosis in calves also affected by viral intestinal infections, and enhancing the local effect of specific antibodies. The basic components can be complemented with further supportive components, such as vitamins, and excipients, the latter ensuring standard contents of the active components and homogeneity and long shelf life of the final product, and facilitate its administration.

The specific antibodies are prepared from colostrum of cows, or preferably from egg yolks of hens, immunized with antigens of bovine rotavirus (such as the CAMP strain V279), bovine coronavirus (such as the CAMP strain V362) and ETEC pathogenic for calves (such as CAMP strains 6231 through 6135). The semi-products, in this case colostrum and liquid eggs, obtained from the immunized donors are homogenized and preserved by spray, fluid, or freeze drying. The latter technique should be preferred because it preserves fully the biological potential of the input materials. The resulting product is a white or yellowish loose powder. The minimal acceptable neutralization titre of antibodies to rotavirus and coronavirus in the dried colostrum or egg yolk is 64 for 100 TCID₅₀ (50% Tissue Culture Infectious Dose) per 1g dried product.

The agglutination titre of antibodies to ETEC must reach at least 80 x 10⁸ inactivated bacterial cells per 1 g dried product.

The other active component of the product are stabilized live cultures of selected lactacidogenic bacterial strains, for example of *Enterococcus* spp and/or *Lactobacillus* spp, such as *Enterococcus faecium* strain M74 - CCM 6226; *Lactobacillus casei* strain CCM 3775, or *Lactobacillus plantarum* strain CCM 3769.

The probiotic component of the product can be obtained by submersive pulsative culture of the respective strains. The bacterial mass resulting from the exponential growth phase is separated from the medium, supplemented with a cryoprotective agent and stabilized by freeze-drying. The concentration of live cells of lactoacidogenic bacteria shall reach at least 5 x 10⁵ CFU (Colony Forming Units) per 1 g dry probiotic concentrate.

In order to increase the metabolic rate and enhance the resistance of the organism to infection, it is recommendable to supplement the product with vitamins A, D₃ and E concentrates in dry, oil or water-soluble form. The minimal daily doses can be **1000** IU vitamin A, **100** IU vitamin D₃ and 3 mg vitamin E per animal.

The product is intended exclusively for oral administration to calves of various ages and therefore a paste form and a powder form have been devised.

The paste is intended above all for oral administration to newborn and suckled calves. The product is packaged in special plastic applicators allowing accurate and reliable dosage without any risk of aspiration.

The water-soluble powder form (pulvis, premix) allows both individual dosage with a measuring jar and group dosage of the product mixed into skimmed milk, milk replacers or other milk-based combined feeds throughout the rearing period. The powder form is also suitable for mixing into rehydration solutions and other product intended for symptomatic therapy of infectious diarrhoea in calves.

The major excipient components of the paste are colloidal silicone hydrolysate and distilled monoglycerides that along with quality edible oil form a consistence with a molecular mesh ensuring a uniform dispersion of the active components. The personnel involved in paste processing must adhere to rules of aseptic work in all operations. Dried skimmed milk and dried whey combined with saccharose, glucose, sorbitol and lactose are used as excipients in the powder formula. Also provided according to the present invention is a method of manufacture of a product according to the present invention, characterised by collecting from cows and/or hens immunised with antigens of bovine rotavirus, bovine coronavirus and enterotoxigenic strains of *Escherichia coli*, colostrum from the immunised cows and/or egg yolks from the immunised hens, processing of these semi-products into the administration form, for instance by drying, and mixing with stabilised live culture of lactacidogenic bacteria.

Strains CAMP V-279, CAMP V-352 and CAMP 6231 through 6235 may be used as the antigen.

The major benefit of the oral product for the prevention and treatment of infectious gastroenteritis of calves is the fact that local immunity of the gastrointestinal tract against combined infections is induced by combined action of antibodies to rotaviruses, coronaviruses and ETEC and the inhibition effect of lactacidogenic bacteria against enteropathogenic and enterotoxigenic bacteria. Thus the product of the present invention eliminates the drawbacks of the current preventive and therapeutic methods without affecting their efficacy. On the contrary, the induction of local immunity by the product enhances the vaccination effect and enhances the natural development of general immunity.

The product can be administered to any age group and particularly to newborn calves that are at the highest risk in the contaminated environment. Another advantage of the product is its composition, because it is produced from biological substances with nutritional characteristics identical with those of common components of calf feeds. The colostral and egg yolk antibodies as active components of the product are resistant to low pH and proteolytic enzymes and penetrate to the site of infection without harm. The paste, powder and premix forms allow optimal ways of administration to calves of any age categories.

### Examples of embodiments

The product for oral prevention and treatment of infectious gastroenteritides of calves is composed of the two basic active components, excipients and vitamin supplements processed into two administration forms, i.e. paste and powder.

The paste is intended for direct individual administration to calves, particularly newborn ones, while the water-soluble powder (pulvis or premix) is added to any type of calf feeds allowing both individual and group prevention and treatment.

Antibody donor animals (highly pregnant cows or hens) are immunized with antigens of bovine rotavirus (such as the CAPM strain V-279), bovine coronavirus (such as the CAPM strain V-352) and enterotoxigenic ETEC strains carrying the K 99 antigen (such as the CAPM strains 6231 through 6235) maintained in the Collection of Animal Pathogenic Microorganisms, Hudcova 70, 621 32 Brno, Czech Republic).

Selected bovine rotavirus and coronavirus strains are propagated in susceptible cell lines (such as MDBK), and the antigens are concentrated by centrifugation and/or ultracentrifugation. Individual ETEC strains are propagated in peptoner broth, yeast extract or another suitable growth medium. At the end of the exponential growth phase, bacterial cells are separated from the medium and resuspended in physiological saline to obtain a standard concentration such as 10⁴ per 1 ml. The viral and bacterial antigens are mixed, for example at a ratio of 20% rotavirus, 20% coronavirus and 60% ETEC, with an oil adjuvant and administered to pregnant cows in two doses before the expected parturition and/or repeatedly to lying hens at 21-day intervals for a period necessary for the production of specific antibodies.

The titres of the antibodies to rotavirus or coronavirus are checked using virus neutralization test and expressed in terms of reciprocal values of the dilution of colostrum or egg yolk suspension that neutralized 100 TCID₅₀ (50% Tissue Culture Infective Dose) of the respective virus. The titre of the antibodies to enterotoxigenic strains is expressed in terms of a reciprocal value of the colostrum and/or egg suspension dilution that, after mixing with the bacterial antigen, agglutinated 10⁸ bacterial cells.

The materials collected from the immunized donor animals, i.e. colostrum and/or egg yolk suspension, are thoroughly homogenized and preserved by spray, fluid or freeze drying. The latter technique is preferred owing to its having the least effects on biological properties of the processed materials. The resulting yellowish powder is stored in a dry and cold room until further processing.

The most suitable material for the production of the concentrate of lactacidogenic bacteria are strains adhering well to the intestinal mucosa of calves and inhibiting markedly a wide range of enteropathogenic and enterotoxigenic microbes. Strains of *Enterococcus faecium* (such as M 74, CCM 6226), *Lactobacillus casei* (such as CCM 3775) or *Lactobacillus plantarum* (such as CCM 3769), maintained in the Czech Collection of Microorganisms (Tvrdého 14, 602 00, Brno, Czech Republic), are used in the product of the present invention. The best method for obtaining the bacterial mass is submersive pulsative culture ensuring a high production of bacteria in the optimal log growth phase. The harvested bacterial cells are separated from the medium, for instance by centrifugation, and stabilized by freeze-drying. The dry bacterial mass with a minimum concentration of 50 x 10⁹ cells of lactacidogenic bacteria per 1 g can be stored at -18 °C for prolonged periods. Before mixing with the other active components and excipients, this semi-product is warmed to room temperature. Data on packaging, administration and dosage of the product of the present invention are given in the following examples:

### Example 1

Paste is the most suitable and most effective formu to be used in young calves, allowing easy and accurate dosing and avoiding the risk of aspiration of the product. Active components, including antibodies to rotavirus, coronavirus and ETEC, and the concentrate of lactacidogenic bacteria are mixed in the first step. A homogenous mixture of freeze-dried (dried) colostrum and/or freeze-dried (dried) egg yolks collected from the immunized donors, freeze-dried concentrates of cultures of *Enterococcus faecium* and/or *Lactobacillus casei*/*Lactobacillus plantarum* and vitamins A, D₃ and E is prepared. In the next step, this mixture is stepwise added into a homogenous mixture of excipients including quality edible oil, such as ground-nut oil, colloid silod hydrolysate, such as CABOSIL, and distilled monoglycerides, such as Myverol. All the active, supplement and excipient components are processed in a vacuum homogenizer and filled under aseptic conditions into plastic applicators with a volume of 40 or 80 ml. The mean oral daily dose of the paste is 1 to 5 ml per animal.

**Table 1:**

| Basic composition of the product in paste form | |
|---|---|
| **Carrier components** | **w/w%** |
| Quality edible oil (such as ground-nut oil) | 40 - 60 |
| Colloidal siloid (such as CABOSIL) | 4 - 6 |
| Distilled monoglycerides | 1 - 3 |

| **Active components** | |
|---|---|
| Dried colostrum or egg yolks collected from immunized animals | 18 - 40 |
| Probiotic concentrate 200 . 10⁹ CFU per 1 g *Enterococcus faecium, Lactobacillus casei, Lactobacillus plantarum* | 4 - 5 |

| **Vitamin supplement** | |
|---|---|
| Mixture of vitamin A. D₃ and E concentrates | 1 - 3 |

### Example 2

The water-soluble powder form (pulvis, premix) is manufactured in standard blenders (such as Nautamix) with a system of horizontal and vertical mixing. In the first step, the active components including dried colostrum and/or dried egg yolks collected from immunized donor animals, and the freeze dried concentrate of lactacidogenic bacteria, are homogenized with the vitamin A, D₃ and E supplement in a smaller mixer. For the final processing, the mixture is transferred into the large blender containing a smaller amount of the excipient, and only after thorough mixing, the content is completed to the full volume and the mixing is finished. The product of the present invention is packaged into 500-g alufan bags or plastic containers.

In the field, the dosage of the product of the present invention is 5 to 15 g per calf and day, or 500 to 1 000 g per 1 ton dry milk replacer or milk-based combined feed.

**Table 2:**

| Basic composition of the product as per invention in the water-soluble powder (pulvis, premix) form | |
|---|---|
| Carrier components | w/w% |
| Dried instant milk or dried whey | 16 - 35 |
| Glucose, lactose, sorbitol, saccharose, starch | 16 - 35 |
| Active components | |
| Dried colostrum or egg yolks collected from immunized animals | 20 - 60 |
| Probiotic concentrate 50 x 10⁹ CFU per 1 g *Enterococcus faecium*, *Lactobacillus casei*, *Lactobacillus plantarum* | 10 - 15 |
| Vitamin supplement | |
| Mixture of vitamin A. D₃ and E concentrates | |

## Claims

1. Oral product for the prevention and therapy of infectious gastroenteritis in calves **characterised in that** it comprises antibodies to bovine rotavirus, bovine coronavirus and enterotoxigenic strains of Escherichia coli contained in colostrum of immunised cows and/or egg yolks of immunised hens and additionally comprising stabilised live culture of lactacidogenic bacteria, said oral product having a minimum titre of antibodies to rotavirus of 64/100 TCID₅₀ and a minimum agglutination titre of antibodies to enterotoxigenic strains of Escherichia coli of 80/10⁸ inactivated bacterial cells per 1 g, the concentration of the stabilised live cultures of lactacidogenic bacteria being within the range of 5 x 10⁵ to 50 x 10⁹ viable bacterial cells per 1 g.

2. Oral product according to Claim 1, wherein the live stabilised cultures of lactacidogenic bacteria are those of *Enterococcus* spp and/or *Lactobacillus* spp.

3. Oral product according to Claim 1 or 2, additionally comprising vitamins and/or excipients.

4. Oral product according to Claim 3, the minimum concentrations of vitamins A, D₃ and E being 1 000 IU, 100 IU and 3 mg, respectively per calf per day.

5. Oral product according to Claims 1 to 4, comprising 18 to 60 w/w% colostrum of immunised cows and/or egg yolks of immunised hens from a total amount of the product.

6. Oral product according to Claim 3, the excipients comprising edible oil 40 to 60 w/w%; silicone hydrolysate 4 to 6 w/w%; distilled monoglycerides 1 to 3 w/w% from a total amount of the product.

7. Oral product according to Claim 3, the excipients comprising glucose and/or saccharose and/or sorbitol in the final concentration of 16 to 35 w/w%, and dried milk and/or dried whey in the final concentration of 16 to 35 w/w% from a total amount of the product.

8. A method of manufacture of a product according to Claims 1 to 7 **characterised by** collecting from cows and/or hens immunized with antigens of bovine rotavirus, bovine coronavirus and enterotoxigenic strains of *Escherichia coli*, colostrum from the immunised cows and/or egg yolks from the immunised hens, processing of these semi-products into the administration form, for instance by drying, and mixing with a stabilised live culture of lactacidogenic bacteria.

9. A method of manufacture of a product according to claim 8 **characterised by** the use of the strains CAMP V-279, CAMP V-352, and CAMP 6231 through 6235 as the antigen.

## Patentansprüche

1. Orales Produkt für die Prävention und Therapie der infektiösen Gastroenteritis in Kälbern, **dadurch gekennzeichnet, dass** es Antikörper zum bovinen Rotavirus, bovinen Koronavirus und enterotoxigene Stämme von Escherichia coli, die im Kolostrum von immunisierten Kühen und/oder Eidotter von immunisierten Hennen enthalten sind, und zusätzlich stabilisierte Lebendkulturen von lactacidogenen Bakterien enthält, wobei das orale Produkt einen minimalen Titer der Antikörper zum Rotavirus von 64/100 TCID₅₀ und einen minimalen Agglutinations-Titer der Antikörper zu enterotoxigenen Stämmen von Escherichia coli von 80/10⁸ inaktivierten Bakterienzellen pro 1 g und die Konzentration der stabilisierten Lebendkulturen von lactacidogenen Bakterien innerhalb eines Bereiches von 5x10⁵ bis 50x10⁹ lebensfähigen Bakterienzellen pro 1 g liegt.

2. Orales Produkt gemäß Anspruch 1, **dadurch gekennzeichnet, dass** die stabilisierten Lebendkulturen von lactacidogenen Bakterien solche von Enterococcus spp und/oder Lactobacillus spp sind.

3. Orales Produkt gemäß Anspruch 1 oder 2, **dadurch gekennzeichnet, dass** es zusätzlich Vitamine und/oder Arzneiträger enthält.

4. Orales Produkt gemäß Anspruch 3, **dadurch gekennzeichnet, dass** die minimalen Konzentrationen von den Vitaminen A, D₃ und E pro Kalb pro Tag 1000 IU, 100 IU bzw. 3 mg betragen.

5. Orales Produkt gemäß den Ansprüchen 1 bis 4, **dadurch gekennzeichnet, dass** in der Gesamtmenge des Produkts 18 bis 60 Gew.-% Kolostrum von immunisierten Kühen und/oder Eidotter von immunisierten Hennen enthalten sind.

6. Orales Produkt gemäß Anspruch 3, **dadurch gekennzeichnet, dass** die Arzneiträger 40 bis 60 Gew.-% Speiseöl, 4 bis 6 Gew.-% Silikon-Hydrolysat, 1 bis 3 Gew.-% destillierte Monoglyceride, jeweils bezogen auf die Gesamtmenge des Produkts, enthält.

7. Orales Produkt gemäß Anspruch 3, **dadurch gekennzeichnet, dass** die Arzneiträger Glukose und/oder Saccharose und/oder Sorbitol in der Endkonzentration von 16 bis 35 Gew.-%, und getrocknete Milch und/oder getrocknete Molke in der Endkonzentration von 16 bis 35 Gew.-%, jeweils bezogen auf die Gesamtmenge des Produkts, enthalten.

8. Ein Verfahren zur Herstellung eines Produkts gemäß den Ansprüchen 1 bis 7, **gekennzeichnet durch** Sammeln von Kühen und/oder Hühnern, die mit Antigenen zum bovinen Rotavirus, bovinen Koronavirus und enterotoxigenen Stämmen von Escherichia coli immunisiert wurden, von Kolostrum von den immunisierten Kühen und/oder Eidotter von den immunisierten Hühnern, Verarbeitung dieser Halbprodukte in eine Verabreichungsform, z. B. **durch** Trocknung und Mischung mit einer stabilisierten Lebendkultur von lactacidogenen Bakterien.

9. Ein Verfahren zur Herstellung eines Produkts gemäß Anspruch 8, **gekennzeichnet durch** die Verwendung der Stämme CAMP V-279, CAMP V-352, und CAMP 6231 bis 6235 als dem Antigen.

## Revendications

1. Produit oral pour la prévention et le traitement d'une gastroentérite infectieuse chez le veau, **caractérisé en ce qu'**il comprend des anticorps dirigés contre le rotavirus bovin, le coronavirus bovin et les souches entérotoxiques d'*Escherichia coli* contenus dans le colostrum de vaches immunisées et/ou dans le jaune d'oeuf de poules immunisées et comprenant en outre une culture vivante stabilisée de bactéries lactacidogènes, ledit produit oral ayant un titre minimum d'anticorps dirigés contre le rotavirus de 64/100 DICT₅₀ et un titre minimal d'agglutination des anticorps dirigés contre les souches entérotoxiques d'*Escherichia coli* de 80/10⁸ cellules bactériennes inactivées pour 1 gramme, la concentration des cultures vivantes stabilisées de bactéries lactacidogènes étant dans l'intervalle de 5 × 10⁵ à 50 × 10⁹ cellules bactériennes viables pour 1 gramme.

2. Produit oral selon la revendication 1, dans lequel les cultures vivantes stabilisées de bactéries lactacidogènes sont celles *d'Enterococcus* spp et/ou de *Lactobacillus* spp.

3. Produit oral selon la revendication 1 ou 2, comprenant en outre des vitamines et/ou des excipients.

4. Produit oral selon la revendication 3, les concentrations minimales en vitamines A, D₃ et E étant de 1 000 UI, 100 UI et 3 mg, respectivement, par veau par jour.

5. Produit oral selon les revendications 1 à 4, comprenant 18 à 60 % en masse/masse de colostrum de vaches immunisées et/ou de jaune d'oeuf de poules immunisées par rapport à la quantité totale de produit.

6. Produit oral selon la revendication 3, les excipients comprenant 40 à 60 % en masse/masse d'huile comestible, 4 à 6 % en masse/masse d'un hydrolysat de silicone ; 1 à 3 % en masse/masse de monoglycérides distillés par rapport à la quantité totale de produit.

7. Produit oral selon la revendication 3, les excipients comprenant du glucose et/ou du saccharose et/ou du sorbitol à une concentration finale de 16 à 35 % en masse/masse et du lait déshydraté et/ou du lactosérum déshydraté à une concentration finale de 16 à 35 % en masse/masse par rapport à la quantité totale de produit.

8. Méthode de fabrication d'un produit selon les revendications 1 à 7, **caractérisée par** le recueil, chez des vaches et/ou des poules immunisées par des antigènes de rotavirus bovin, de coronavirus bovin et de souches entérotoxiques d'*Escherichia coli,* du colostrum des vaches immunisées et/ou du jaune d'oeuf des poules immunisées, la transformation de ces semi-produits en une forme d'administration, par exemple par séchage, et le mélange avec une culture vivante stabilisée de bactéries lactacidogènes.

9. Méthode de fabrication d'un produit selon la revendication 8, **caractérisée par** l'utilisation des souches CAMP V-279, CAMP V-352 et CAMP 6231 à 6235 comme antigènes.
